# EUROPÄISCHE PATENTSCHRIFT

(11) **EP 0 733 702 B1**
(45) Veröffentlichungstag und Bekanntmachung des Hinweises auf die Patenterteilung: **07.05.2003**
(21) Anmeldenummer: 96101959.3
(22) Anmeldetag: 10.02.1996
(51) Int. Cl.: C12N 9/10, A61K 38/36

(54) **Stabile Transglutaminasepräparate und Verfahren zu ihrer Herstellung**
Stable transglutaminase preparations and process for their preparation
Préparations stables de transglutaminase et procédé de leur préparation

(30) Priorität: 09.03.1995 DE 19508192
(43) Veröffentlichungstag der Anmeldung: 25.09.1996
(73) Patentinhaber: Aventis Behring Gesellschaft mit beschränkter Haftung, 35002 Marburg (DE)
(72) Erfinder: Metzner, Hubert, Dr., D-35094 Lahntal (DE); Karges, Hermann, Dr., D-35041 Marburg (DE)

(56) Entgegenhaltungen:
- EP-A- 0 018 561
- EP-A- 0 037 078
- EP-A- 0 637 451
- WO-A-92/00767
- WO-A-93/15234

## Beschreibung

Gegenstand der vorliegenden Erfindung sind stabile Transglutaminasepräparate, insbesondere stabile Faktor XIII-Präparate, und Verfahren zu ihrer Herstellung.

Faktor XIII (F XIII, Fibrin-stabilisierender Faktor), eine in Plasma, Plättchen und Monozyten/Makrophagen als Proenzym vorkommende Transglutaminase, ist u.a. für eine ungestörte Blutgerinnung und Wundheilung von Bedeutung. F XIII wird in Form von frisch gefrorenem Plasma, isoliert aus Plazenta oder Plasma therapeutisch schon über Jahre hinweg mit gutem Erfolg für die Therapie des Faktor XIII-Mangels eingesetzt. Mittlerweile ist auch die rekombinante Herstellung von Faktor XIII (rF XIII) möglich.

Die kommerziell angebotenen, gereinigten oder teilgereinigten Transglutaminase- bzw. F XIII-Präparate enthalten zugesetzte Stabilisatoren wie humanes Serumalbumin (HSA), was aber in Hinblick auf die damit verbundene Verminderung der spezifischen Aktivität, die zusätzliche Proteinbelastung bzw. potentielle Immunogenität sowie die Beeinträchtigung der Reinheitsbeurteilung für Proteinpräparate von Nachteil ist. Besonders bei hochreinen Proteinen (wie z.B. rekombinanten Proteinen) ist es wünschenswert, die erreichte Reinheit nicht wieder durch Zusatz von Fremdproteinen zu reduzieren. Ferner ist durch den Zusatz von z.B. Albumin auch eine potentielle Belastung mit Virusantigenen gegeben.

Therapeutisch einsetzbare Proteinpräparate müssen in aller Regel über einen längeren Zeitraum hinsichtlich ihrer Zusammensetzung und Aktivität stabil sein. Da dies in Lösung nur selten erreichbar ist, werden solche Produkte häufig getrocknet in den Handel gebracht. Zur Trocknung solcher Produkte ist die schonende Gefriertrocknung die Methode der Wahl. Doch auch bei dieser Methode werden nur unter bestimmten Voraussetzungen stabile Präparationen erhalten, die die Anforderungen an Integrität und Haltbarkeit erfüllen.

Da die Gefriertrocknung von z.B. unstabilisierten Transglutaminase-Lösungen zu einem starken Aktivitätsabfall und zu erheblichen Trübungen führt, sind z.B. für gereinigte F XIII-Präparate bisher Formulierungen auf Basis von Albumin mit mehr oder weniger hohen Konzentrationen von Salzen beschrieben worden (DE-PS 2063 070, JP 53/59018). Diese Formulierungen haben aber den bereits beschriebenen Nachteil des Fremdproteinzusatzes mit all seinen Problemen.

Ferner wurde die Gefriertrocknung von rF XIII in Gegenwart von Glycin oder Arginin und nicht-reduzierenden Zuckern beschrieben (WO 93/03147). Hier wurden aber keine Aussagen über die Stabilität und Löslichkeit bzw. die Klarheit des rekonstituierten Lyophilisates gemacht. Auch wurde das erhaltene Produkt bei -20°C gelagert, vermutlich um aufgrund einer unzureichenden Stabilität bei 4°C das Material stabil zu halten.

Der vorliegenden Erfindung liegt daher die Aufgabe zugrunde, durch eine geeignete Formulierung für Transglutaminasen, insbesondere für F XIII, als lokal (z.B. auch topisch) oder parenteral applizierbares Protein ein bei 2-8°C (oder höher) stabiles, lagerfähiges Produkt zu erhalten, bei dem auf den Zusatz von z.B. HSA verzichtet werden kann. Ferner sollte das Lyophilisat gut löslich sein und nach dem Einlösen eine klare Lösung ohne Trübungen ergeben, die weiterhin noch eine ausreichende Stabilität besitzen sollte.

Diese Aufgabe wurde durch die Bereitstellung von stabilen Zubereitungsformen für Transglutaminasepräparate unter Verwendung bestimmter Stabilisatoren bzw. Gemische davon, wie sie nachfolgend näher beschrieben sind, und von Verfahren zu ihrer Herstellung gelöst. Die Erfindung betrifft somit eine stabile Zubereitungsform einer Transglutaminase, die nach Lyophilisieren gut und ohne Trübung löslich ist, enthaltend die gereinigte Transglutaminase sowie D-und/oder L-Aminosäuren, außer Glycin und Arginin, deren Salze, Derivate und Homologe, Dimere oder Oligomere davon oder Mischungen davon und/oder Zucker oder Zuckeralkohole, gegebenenfalls in Kombination mit ober-flächenaktiven Agentien und/oder reduzierenden Agentien.

Die beispielhaften Ausführungen zeigen dies anhand von rekombinantem Faktor XIII bzw. des aus Plazenta oder Plasma isolierten F XIII, sind jedoch nicht darauf beschränkt. In einer bevorzugten Ausführungsform betrifft die vorliegende Erfindung somit stabile Zubereitungsformen von Faktor XIII, biologisch aktiven Fragmenten, Derivaten oder Muteinen davon.

In einer besonders bevorzugten Ausführungsform handelt es sich bei den stabilen Zubereitungsformen um F XIII aus Plasma, Plazenta, Thrombozyten, Makrophagen/Monozyten oder rekombinanten F XIII enthaltende Zubereitungsformen.

Die erfindungsgemäßen Untersuchungen lassen sich im wesentlichen in zwei Bereiche aufteilen: (a) Untersuchungen zur eigentlichen Gefriertrocknung und (b) zur anschließenden Lagerstabilität.

Für die Untersuchungen wurde rF XIII (Metzner et al., in J. McDonagh, R. Seitz, R. Egbring: "Factor XIII", 87 - 93, Schattauer (1993)), sowie plazentärer und plasmatischer F XIII eingesetzt (Karges u. Rapp, in J. McDonagh, R. Seitz, R. Egbring: "Factor XIII", 66 - 76, Schattauer (1993)).

Die Gefriertrocknungsversuche wurden in handelsüblichen Anlagen unter Verwendung von Glasfläschchen ohne bzw. mit silikonisierter Oberfläche durchgeführt.
(a) Gefriertrocknung:
   Bei dem Vergleich verschiedener Additive wurde überraschenderweise gefunden, daß bei Verwendung von bestimmten Zusätzen aus der Gruppe der D- und/oder L-Aminosäuren, deren Salze, Derivate oder Homologe die Aktivität und Löslichkeit des F XIII bei der Gefriertrocknung teilweise sehr gut erhalten werden kann (Tabelle I).
   In einer weiteren bevorzugten Ausführungsform betrifft die vorliegende Erfindung daher stabile Zubereitungsformen einer Transglutaminase, die die Aminosäuren His, Glu, Met, Thr, Lys, Ala, Ile, Cys, deren Salze, Derivate, Homologe, Dimere oder Oligomere davon, oder Mischungen davon enthalten.
   Speziell die Aminosäuren His und Glu zeigten überraschenderweise bereits ohne weitere Zusätze eine gute Stabilisierung bei der Gefriertrocknung. Bei alleinigem Einsatz von Aminosäuren wie z.B. Gly, Met oder Ala kam es hingegen bei der Gefriertrocknung zu einem deutlichen Aktivitätsabfall (Tabelle I).
   Der alleinige Einsatz von Zuckern oder Zuckeralkoholen ergab teilweise bereits eine gute Stabilisierung im Verlauf der Gefriertrocknung (Tabelle I). Besonders Zucker oder Zuckeralkohole wie Saccharose, Trehalose, Laktose, Maltose, Sorbit, Mannit o.ä. zeigten positive Ergebnisse. Der Aktivitätsabfall bei der Gefriertrocknung mit Aminosäuren als Zusatz, die alleine nicht ausreichend wirksam waren (wie z.B. Met, Ala, u.a.), läßt sich durch Kombination mit Zuckern oder Zuckeralkoholen stark reduzieren (Tabelle I).
   In einer weiteren Ausführungsform betrifft die vorliegende Erfindung deshalb stabile Zubereitungsformen einer Transglutaminase, die die Zucker oder Zuckeralkohole Saccharose, Lactose, Trehalose, Maltose, Sorbit oder Mannit, deren Derivate, Homologe oder Mischungen davon enthalten.
   Der alleinige Einsatz von Puffersubstanzen wie Tris oder Phosphat führte zu keiner nennenswerten Stabilisierung. Allerdings führte der Einsatz von Boraten zu einer deutlichen Stabilisierung im Verlauf der Gefriertrocknung (Tabelle I).
   Ein bei Einlösen des Lyophilisates teilweise auftretender, geringfügiger Proteinniederschlag ließ sich durch Einsatz von oberflächenaktiven Stoffen wie Tween 80 oder Tween 20, Polyethylenglykol (PEG) mit Molekulargewichten zwischen 1000 und 35000 Da, Cetylalkohol, Polyvinylpyrrolidon (PVP), Polyvinylalkohol (PVA), Lanolinalkohol, Sorbitanmonooleat u.a. ohne Aktivitätsverlust des F XIII bei der Gefriertrocknung verhindern (Tabelle II u. III).
   In einer weiteren bevorzugten Ausführungsform enthalten die stabilen Zubereitungsformen als oberflächenaktiven Stoff somit Tween 80, Tween 20, PEG, Cetylalkohol, PVP, PVA, Lanolinalkohol oder Sorbitanmonooleat.
(b) Lagerstabilität:
   Ein kritischer Parameter des formulierten Lyophilisates ist die Lagerstabilität, die bei 4°C und bei Raumtemperatur, unter akzelerierten Bedingungen aber auch bei 37°C, bestimmt wurde.
   Es zeigte sich, daß für eine gute Lagerstabilität die Kombination von Aminosäuren mit Zuckern, Zuckeralkoholen oder Zuckerderivaten vorteilhaft ist (Tabelle IV). Die Untersuchung verschiedener Zucker ergab, daß Zucker wie Saccharose, Lactose, Trehalose oder Maltose die Aktivität auch bei längerer Lagerzeit unter erhöhter Temperatur stabilisieren, während Zucker wie Glucose oder Fructose als reduzierende Zucker bei 37°C einen langsamen Aktivitätsabfall nicht ausreichend verhindern können (Tabelle V). Auch die Kombination der Aminosäuren His oder Glu, die alleine bereits eine gute Stabilität bedingen, mit Zuckern führte zu einer noch weiter erhöhten Stabilität.
   In einer weiteren bevorzugten Ausführungsform enthalten somit die stabilen Zubereitungsformen als Stabilisator Saccharose, Maltose, Trehalose, Lactose, Sorbit oder Mannit, deren Derivate, Homologe oder Mischungen davon, in Kombination mit der Aminosäure His, Glu, Ile und/oder Ala.
   Oberflächenaktive Substanzen zeigten im geeigneten Konzentrationsbereich, wie bereits erwähnt, keine negativen Einflüsse auf die Lagerstabilität (Tabelle II u. III).
   Obwohl F XIII keine zugänglichen SH-Gruppen aufweist, zeigen SH-Agentien wie Cys, N-Acetylcystein, Thioglycerin oder Glutathion besonders bei erhöhten Temperaturen überraschenderweise einen positiven Einfluß auf die Lagerstabilität von F XIII. Komplexbildner wie z.B. EDTA oder Citrat können dabei zum Schutz der SH-Funktionen zugesetzt werden.
   In einer weiteren bevorzugten Ausführungsform enthält die erfindungsgemäße stabile Zubereitungsform einer Transglutaminase daher Cys, N-Acetyl-Cys, Thioglycerin, Natriumsulfid oder Glutathion oder Mischungen davon, ggf. in Anwesenheit eines Komplexbildners.
   Sehr gute Ergebnisse bezüglich Aktivitätserhaltung und Löslichkeit des Lyophilisates ließen sich mit ternären oder quaternären Mischungen (Aminosäure(n), Zucker, oberflächenaktive Komponente) erzielen, beispielsweise mit His/Tween/Saccharose-, His/PEG/Saccharose- oder His/Ile/PEG/Saccharose-Mischungen. In einer weiteren bevorzugten Ausführungsform enthält die erfindungsgemäße stabile Zubereitungsform einer Transglutaminase als Zusätze somit außer einer Aminosäure(n) einen Zucker oder Zuckeralkohol und eine oberflächenaktive Substanz, besonders bevorzugt sind dabei die Zusatzmischungen His/Tween 20/Saccharose oder His/Tween 80/Saccharose, His/PEG/Saccharose oder His/Ile/PEG/Saccharose.
   In Bezug auf die Lagerstabilität erwiesen sich Mischungen aus Aminosäure(n) und/oder Zucker bzw. Zuckeralkohol, einer oberflächenaktiven Substanz und einem reduzierenden Agens, z.B. Mischungen von Aminosäure(n)/Cys bzw. N-Acetyl-Cys/PEG/Saccharose, Aminosäure(n)/Thiogly-cerin/PEG/Saccharose und Zucker/ Reduktionsmittel/PEG ebenfalls als ein geeignetes Formulierungssystem, besonders auch bei höheren Temperaturen (siehe Tabelle III und VI). In einer weiteren bevorzugten Ausführungsform enthält daher die erfindungsgemäße Zubereitungsform als Zusatz eine Mischung aus einer Aminosäure(n) und/oder Zucker bzw. Zuckeralkohol, einer oberflächenaktiven Substanz und einem reduzierenden Agens, besonders bevorzugt sind die Mischungen Aminosäure(n)/Cys/PEG/Saccharose, Aminosäure(n) / N-Acetyl-Cys/PEG/Saccharose, Aminosäure(n) / Thioglycerin/PEG/Saccharose und Zucker/Reduktionsmittel/PEG, ggf. in Anwesenheit eines Komplexbildners.
   Bei den beschriebenen Formulierungen ist die Konzentration des eingesetzten F XIII in einem weiten Bereich variierbar und liegt vorzugsweise im Bereich von 0,003-50 mg/ml.
   Die Konzentrationen der eingesetzten Aminosäuren liegen vorzugsweise in einem Bereich von 0,01-10% (Gew./V.), besonders aber in einem Bereich von 0,1-3% (Gew./V.). Die Zuckerkonzentrationen liegen vorzugsweise bei 0,1-20% (Gew./V.), besonders bevorzugt aber zwischen 0,2-10% (Gew.N.). Oberflächenaktive Komponenten sind vorzugsweise in einem Konzentrationsbereich von 0,00001-5% (Gew./V.) einsetzbar. besonders zwischen 0,0002% und 0,1%. Die Konzentrationen der reduzierenden Agentien liegen vorzugsweise zwischen 0,001% und 2% (Gew.N.), besonders aber zwischen 0,005% und 0,5%.
   Für die Lagerstabilität des lyophilisierten Proteins ist auch die Restfeuchte von Bedeutung. Mit den angegebenen Zusätzen lassen sich in einer Schlußphase der Gefriertrocknung die Temperaturen für mehrere Stunden ohne Aktivitätsverlust auf 50-60°C erhöhen, soweit dies für die Reduktion der Restfeuchte notwendig ist.
   Für die Gefriertrocknung von Transglutaminasen bzw. F XIII sowie für die anschließende Lagerstabilität sollte der pH-Wert der Lösungen vorzugsweise in einem Bereich von 6-9, besonders bevorzugt zwischen 7 und 8, liegen. Zur Pufferung eignen sich bevorzugt die eingesetzten Aminosäuren, Phosphatpuffer, Boratpuffer oder Trispuffer mit einem pH-Wert im Bereich von 6 bis 9, die ggf. in Kombination mit einem Komplexbildner verwendet werden.
   Die vorliegende Erfindung umfaßt auch die Verwendung der oben beschriebenen Stabilisierungszusätze zur Herstellung von stabilen, (ein) Protein(e) enthaltenden Flüssigpräparationen, da die durch die beispielhaft an Transglutaminasen durchgeführten Experimente gewonnenen Ergebnisse auf andere Präparate übertragbar sind.
   Die vorliegende Erfindung umfaßt weiterhin ein Verfahren zur Stabilisierung von Proteinen, vorzugsweise Transglutaminasen, bei dem die gereinigten Proteine bzw. das gereinigte Protein, bei dem es sich vorzugsweise um eine Transglutaminase handelt, als Lösung oder Präzipitat mit einer Lösung, die einen oder mehrere der erfindungsgemäßen Zusätze enthält und auf einen für die Stabilität vorteilhaften pH-Bereich eingestellt ist, nach üblicher Vorgehensweise vermischt und danach gefriergetrocknet wird.
   Aufgrund der hervorragenden Eigenschaften der nach dem erfindungsgemäßen Verfahren hergestellten stabilisierten Transglutaminasen eignen sich diese sehr gut zur Formulierung eines Arzneimittels.
   Die vorliegende Erfindung schließt außerdem die Verwendung der Faktor XIII, biologisch aktive Fragmente, Derivate oder Muteine davon enthaltenden stabilen Zubereitungsformen zur Herstellung eines Arzneimittels zur Behandlung von z.B. durch F XIII-Mangel charakterisierten Krankheiten mit ein.
   Die erfindungsgemäßen Arzneimittel können gegebenenfalls mit geeigneten, pharmazeutisch verträglichen, allgemein bekannten Trägern nach bekannten Verfahren formuliert werden. Sie können in geeigneter Dosierung, die vom behandelnden Arzt bestimmt werden kann, verabreicht werden. Die Verabreichung kann auf verschiedenen Wegen erfolgen, z.B. intravenös, intraperitoneal, subkutan, intramuskulär, intradermal oder topisch.
   Die Beispiele erläutern die Erfindung.

### Beispiel 1

rF XIII, hergestellt durch Expression in Hefezellen und mittels geeigneter Verfahren bis zu einem Gehalt von >98% aufgereinigt, sowie der plazentäre F XIII, ebenfalls in gereinigter Form, wurden als Lösung oder als Präzipitat mit Lösungen der Stabilisatoren versetzt, um zu den angegebenen Aktivitäten zu gelangen. Die F XIII-Lösungen wurden filtriert, in Glasfläschchen abgefüllt und nach einem geeigneten Gefriertrocknungsprogramm getrocknet. Anschließend wurden die Lyophilisate wieder mit aqua dest. auf das ursprüngliche Volumen rekonstituiert. Vor und nach der Gefriertrocknung wurden die F XIII-Aktivitäten bestimmt. Ferner wurde die rekonstituierte Lösung hinsichtlich Trübungen beurteilt.
Die Bestimmung der F XIII-Aktivität erfolgte mit Hilfe des kommerziell verfügbaren Berichrom F XIII^{R} Test Kits.

Die in den Tabellen I, II und III dargestellten Ergebnisse zeigen eindeutig, daß der Zusatz der erfindungsgemäßen stabilisierenden Bestandteile bei der Gefriertrocknung von F XIII notwendig ist und daß durch Zugabe von Aminosäuren oder Zuckern bzw. Aminosäuren und Zuckern die enzymatische Aktivität und die Löslichkeit auch ohne die zusätzliche Verwendung von HSA erhalten werden können. Besonders die Löslichkeit läßt sich durch Zusatz von oberflächenaktiven Agentien teilweise noch verbessern.

### Beispiel 2

rF XIII- und Plazenta-F XIII-Lyophilisate, hergestellt wie in Beispiel 1 angegeben, wurden bei 4°C bzw. bei 37°C gelagert und nach verschiedenen Zeiten mit Aqua Injectab. oder mit Kochsalzlösung rekonstituiert, um die verbleibende Enzymaktivität zu bestimmen.

Die in den Tabellen II bis VI dargestellten Ergebnisse zeigen, daß eine ausreichende Langzeitstabilisierung durch die bereits beschriebenen Mischungen, die eine Aminosäure bzw. Aminosäuren und/oder Zucker bzw. Zuckerderivate umfassen, zu erreichen ist. Der Zusatz einer reduzierenden Komponente führt dabei teilweise noch zu einer erhöhten Stabilität, besonders unter akzelerierten Bedingungen.

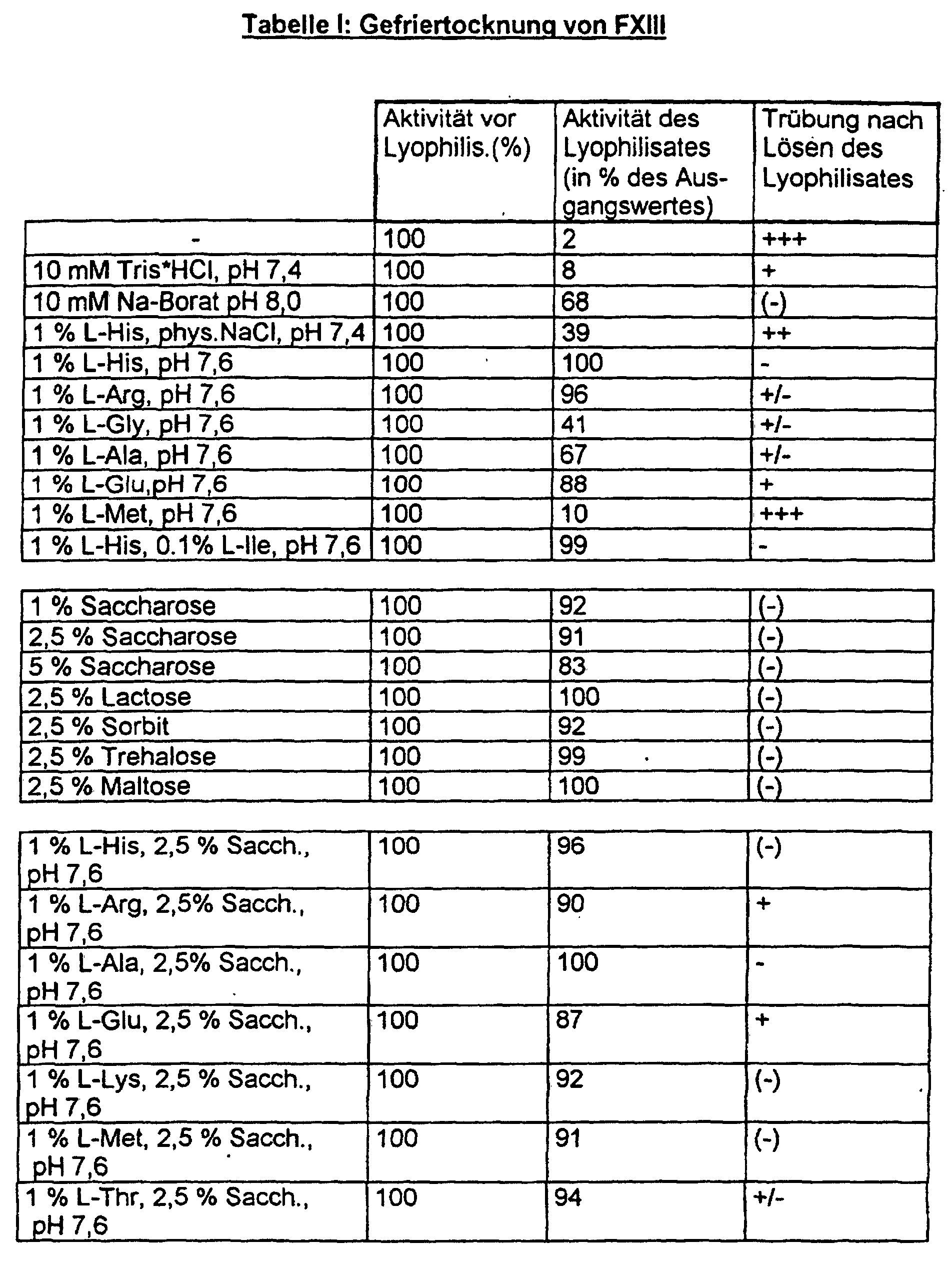

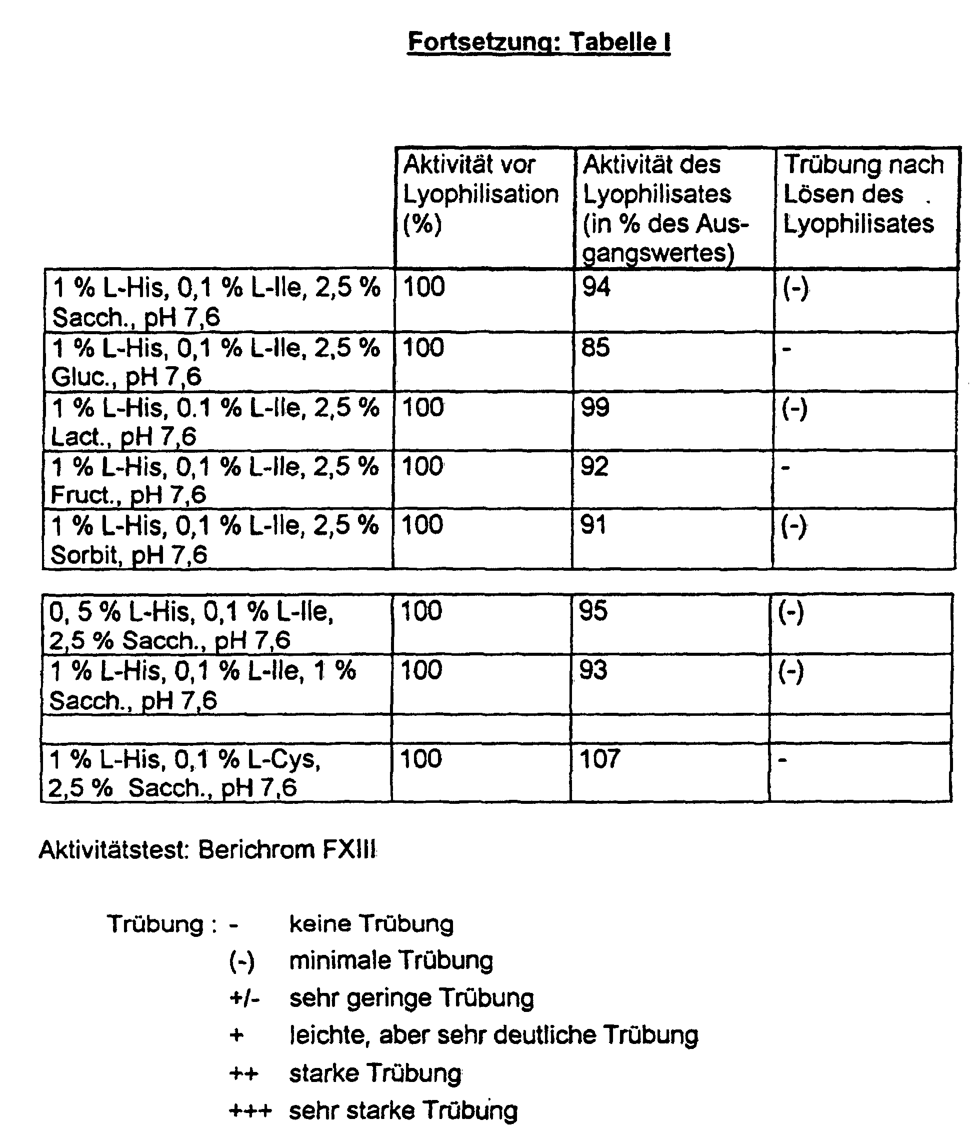

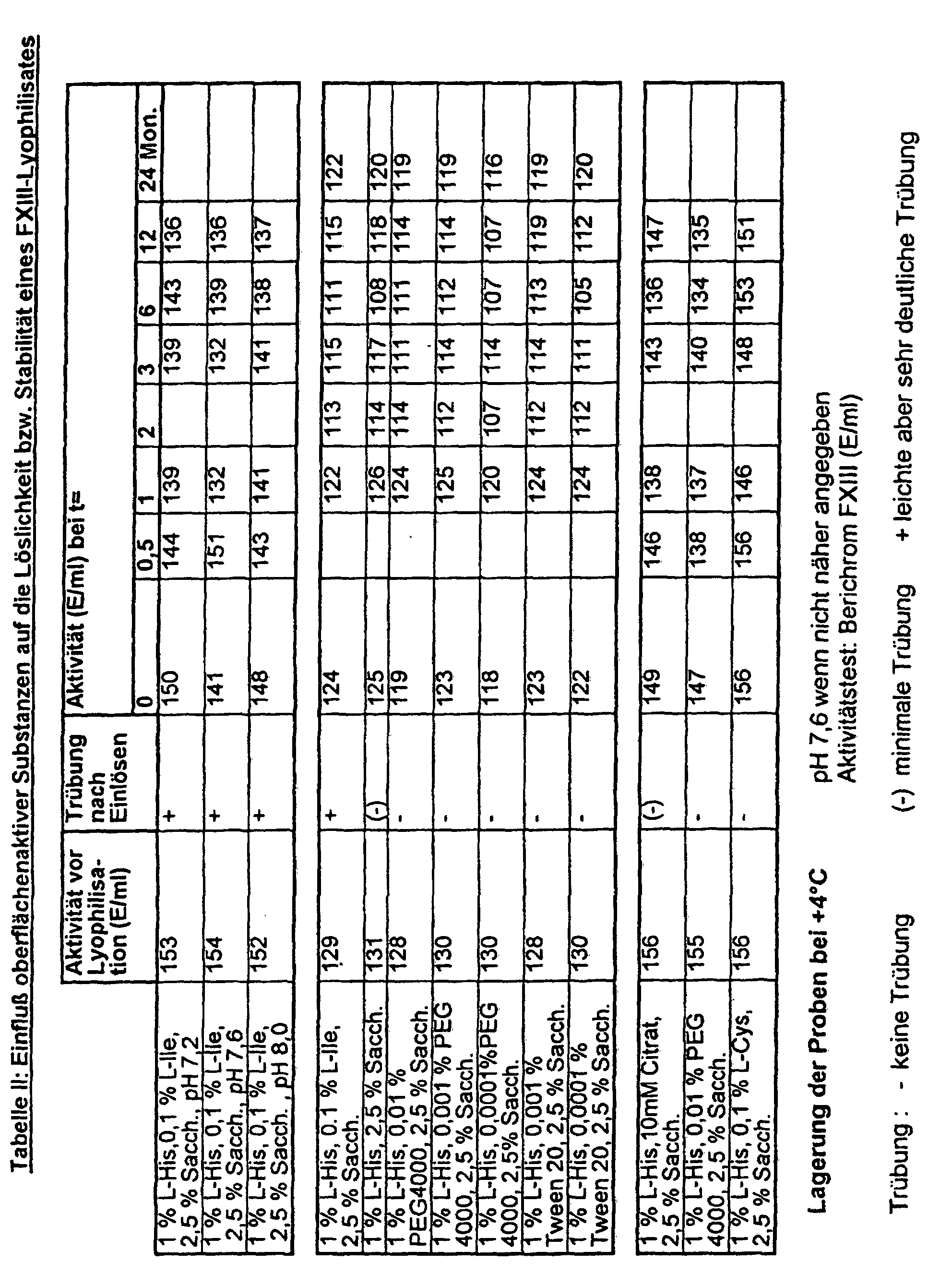

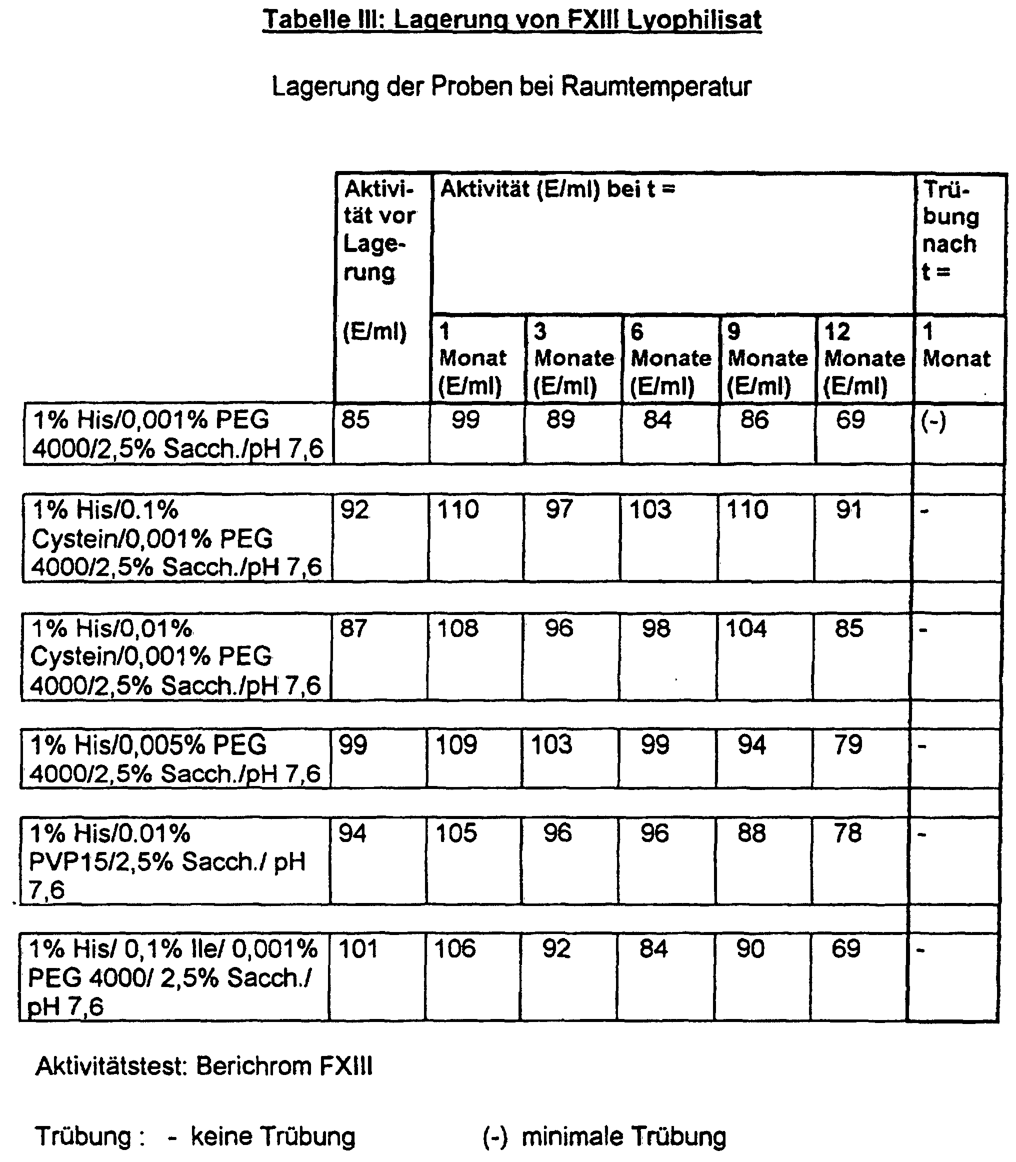

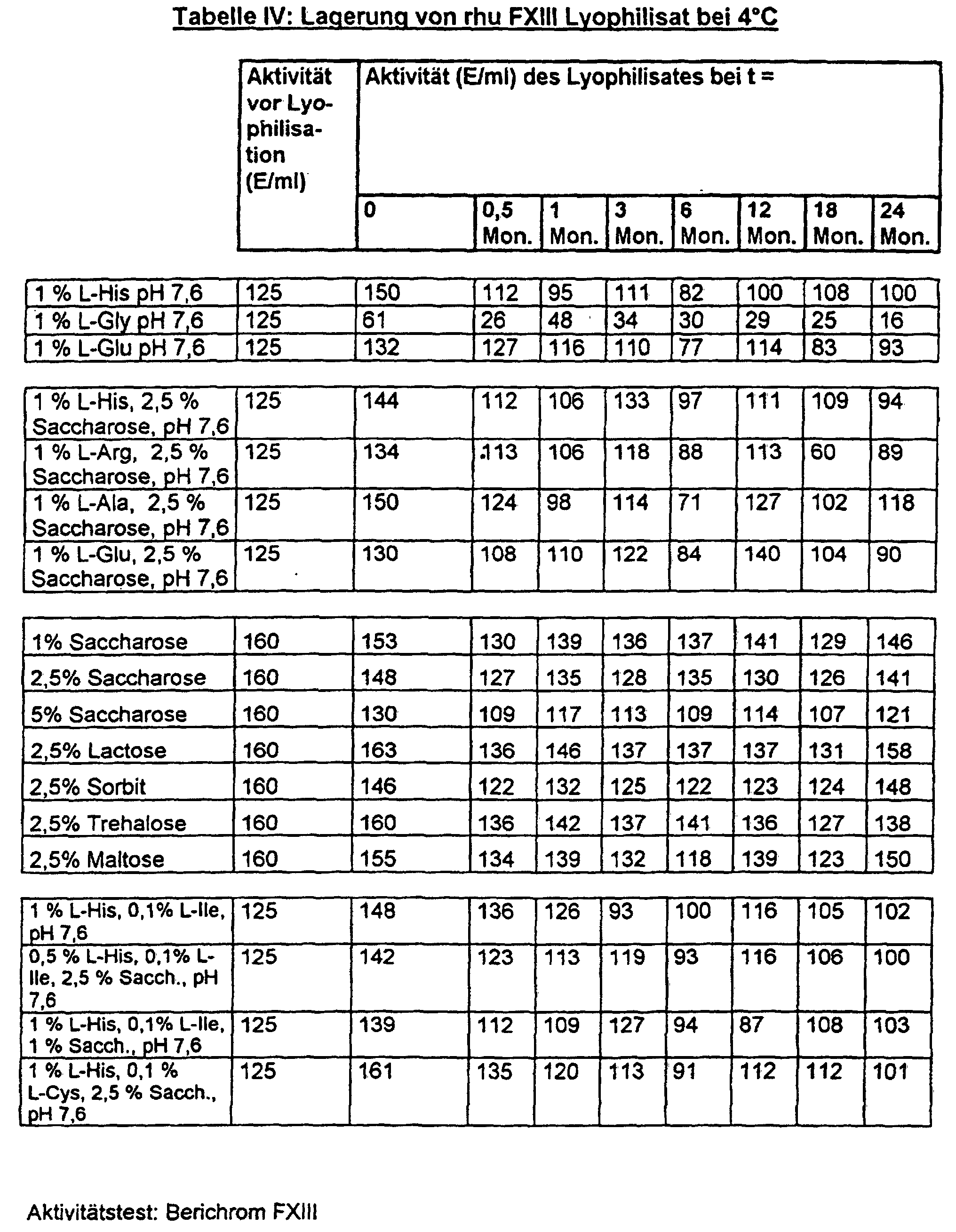

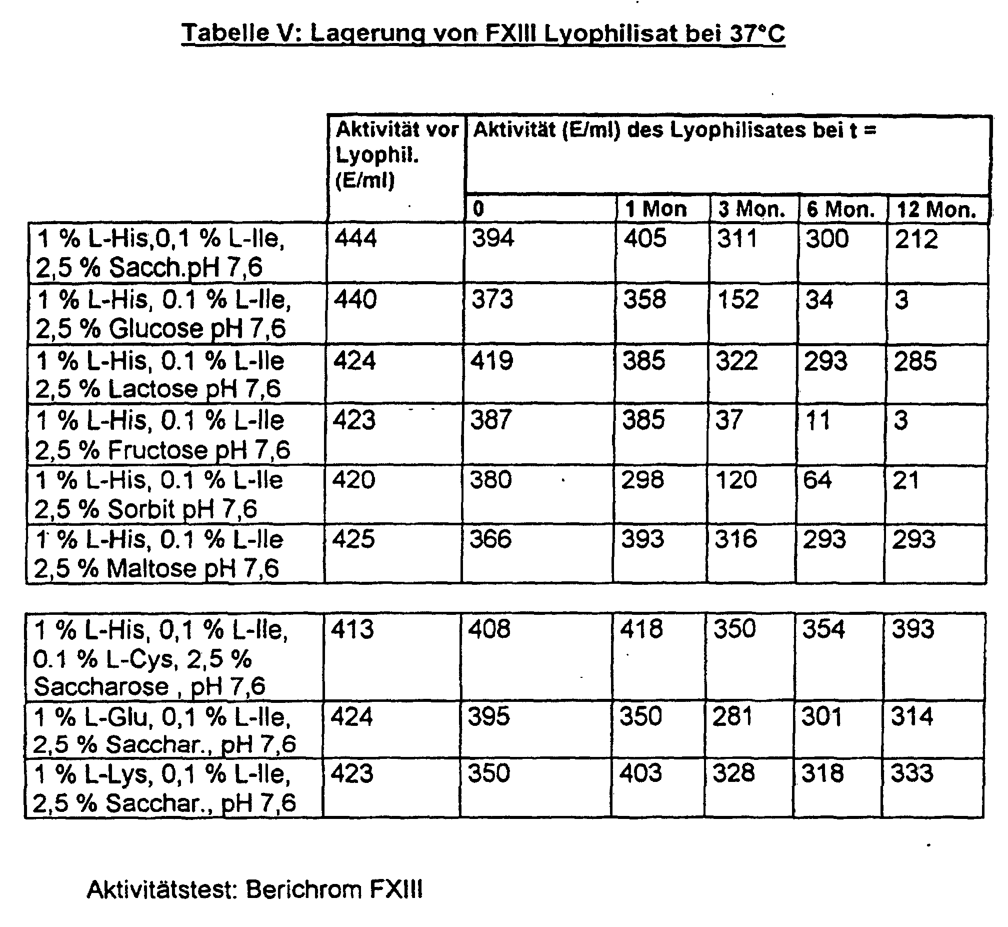

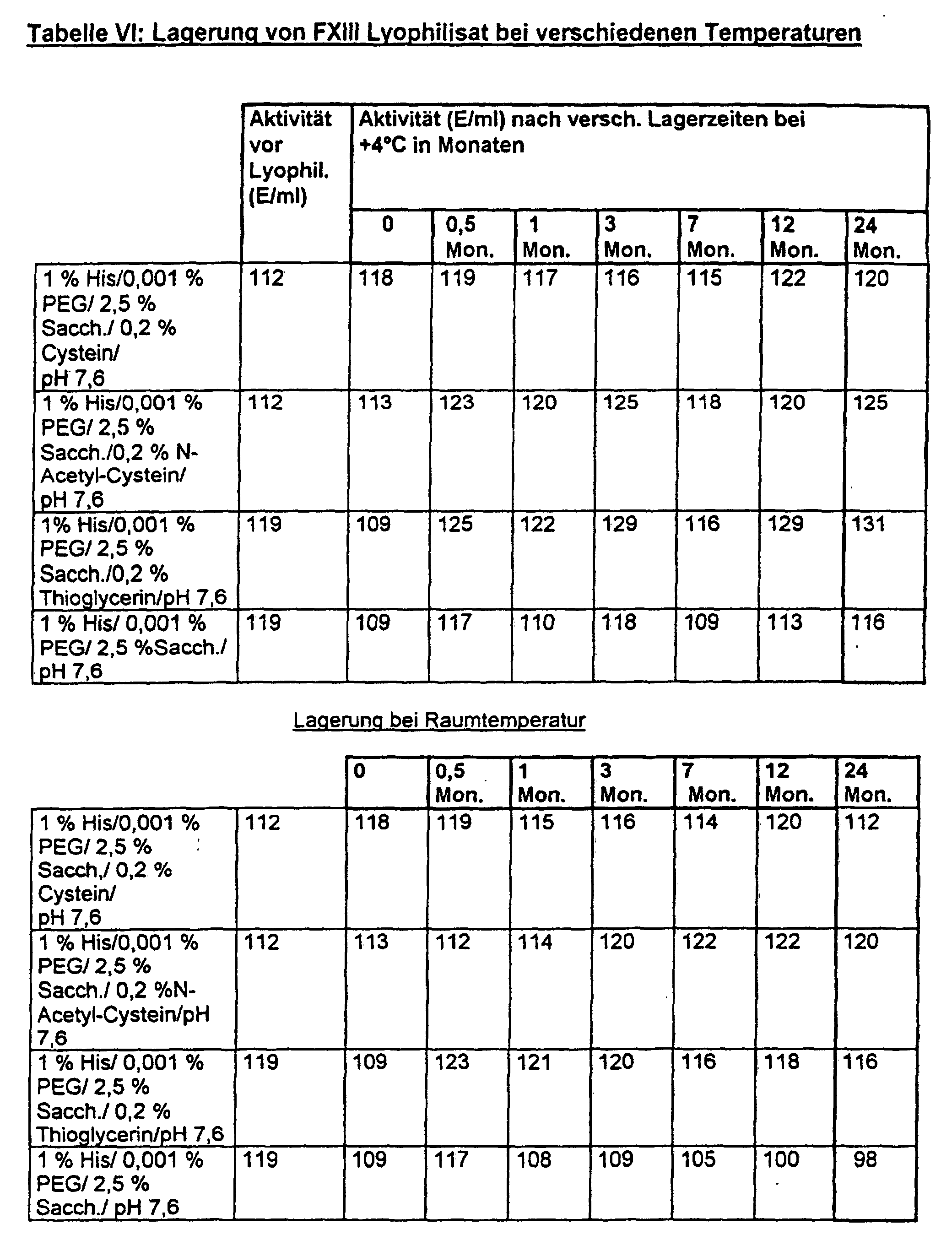

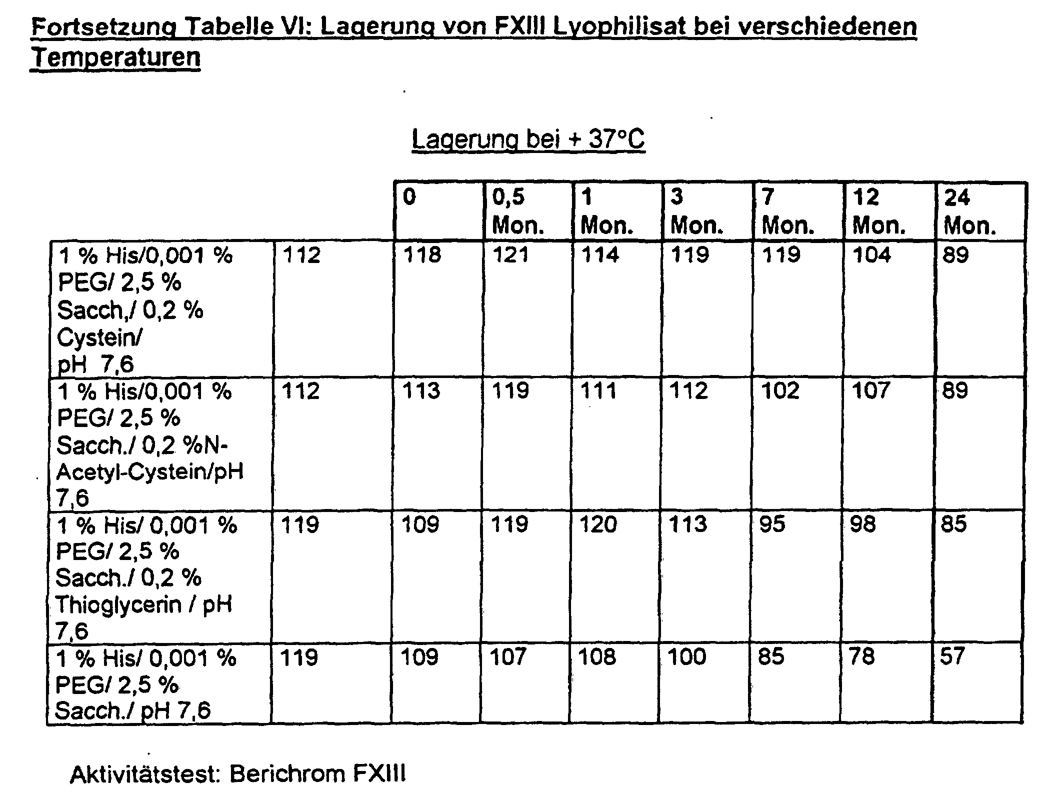

## Patentansprüche

1. Lyophilisierte Zubereitungsform einer Transglutaminase ohne Zusatz von Fremdproteinen, wobei
(i) nach 6 Monaten Lagerung bei 4°C die Aktivität der Transglutaminase noch mehr als 80% der Ausgangsaktivität vor der Lyophilisation beträgt,
(ii) das Lyophilisat nach der Lagerung gut und ohne oder mit minimaler Trübung löslich ist,
(iii) als Zusatz
(a) Zucker oder Zuckeralkohole, oder Mischungen davon,
(b) Histidin alleine oder als Mischung mit Cystein oder Isoleucin, oder
(c) Alanin, Glutaminsäure, Methionin, Cystein oder Histidin alleine oder als Mischung mit Cystein oder Isoleucin als Mischung mit Zucker oder Zuckeralkoholen oder Mischungen davon enthalten sind.

2. Zubereitungsform nach Anspruch 1, die ferner oberflächenaktive Agentien und/oder reduzierende Agentien enthält.

3. Zubereitungsform nach Anspruch 1 oder 2, wobei es sich bei der Transglutaminase um Faktor XIII (F XIII), biologisch aktive Fragmente, Derivate oder Muteine davon handelt.

4. Zubereitungsform nach Anspruch 3, wobei es sich um F XIII aus Plasma, Plazenta, Thrombozyten, Makrophagen/Monozyten oder um rF XIII oder um biologisch aktive Fragmente, Derivate oder Muteine davon handelt.

5. Zubereitungsform nach einem der Ansprüche 1 bis 4, wobei es sich bei dem Zucker oder Zuckeralkohol um Saccharose, Maltose, Trehalose, Lactose, Sorbit oder Mannit, deren Derivate, Homologe oder Mischungen handelt.

6. Zubereitungsform nach Anspruch 1, wobei die Zucker oder Zuckeralkohole in Kombination mit der Aminosäure His, Glu, Ile und/oder Ala vorliegen.

7. Zubereitungsform nach Anspruch 2, wobei es sich bei dem oberflächenaktiven Agens um Tween 80, Tween 20, PEG, Cetylalkohol, PVP; PVA, Lanolinalkohol oder Sorbitanmonooleat handelt.

8. Zubereitungsform nach Anspruche 2, wobei es sich bei dem reduzierenden Agens um Cys, N-Acetyl-Cys, Thioglycerin, Natriumsulfid oder Glutathion oder Mischungen davon handelt, gegebenenfalls in Anwesenheit eines Komplexbildners.

9. Zubereitungsform nach Anspruch 2, die als Zusätze außer einer Aminosäure(n) einen Zucker oder Zuckeralkohol und eine oberflächenaktive Substanz enthält.

10. Zubereitungsform nach Anspruch 9, die als Zusätze His/Tween 80/Saccharose, His/Tween 20/Saccharose, His/PEG/Saccharose oder His/lle/PEG/Saccharose enthält.

11. Zubereitungsform nach Anspruch 2, die als Zusätze (eine) Aminosäure(n) und/oder einen Zucker bzw. Zuckeralkohol, eine oberflächenaktive Substanz und ein reduzierendes Agens enthalten.

12. Zubereitungsform nach Anspruch 11, die als Zusätze (eine) Aminosäure(n)/Cys/PEG/Saccharose, (eine) Aminosäure(n)/N-Acetyl-Cys/PEG/Saccharose, (eine) Aminosäure(n) Thioglycerin/PEG/Saccharose oder Zucker/Reduktionsmittel/PEG enthält, gegebenenfalls in Anwesenheit eines Komplexbildners.

13. Zubereitungsform nach einem der Ansprüche 1 bis 12, wobei die Konzentration der Transglutaminase im Bereich von 0,003 bis 50 mg/ml liegt.

14. Zubereitungsform nach einem der Ansprüche 1 bis 13, wobei die Konzentration der Aminosäuren, deren Salze, Derivate oder Homologe im Bereich von 0,01 bis 10% (Gew./V.), vorzugsweise von 0,1 bis 3% (Gew.N.) liegt.

15. Zubereitungsform nach einem der Ansprüche 1 bis 14, wobei die Konzentration des Zuckers oder Zuckeralkohols zwischen 0,1 und 20% (Gew./V.), vorzugsweise zwischen 0,2 und 10% (Gew.N.) liegt.

16. Zubereitungsform nach einem der Ansprüche 2 bis 15, wobei die Konzentration des oberflächenaktiven Agens zwischen 0,00001 und 5% (Gew./V.), vorzugsweise zwischen 0,0002 bis 0,1% (Gew./V.) liegt.

17. Zubereitungsform nach einem der Ansprüche 2 bis 16, wobei die Konzentration des reduzierenden Agens zwischen 0,001% und 2% (Gew.N.), vorzugsweise zwischen 0,005 und 0,5% (Gew./V.) liegt.

18. Zubereitungsform nach einem der Ansprüche 1 bis 17, wobei der pH-Wert in einem Bereich von 6 bis 9, vorzugsweise zwischen 7 und 8 liegt.

19. Zubereitungsform nach Anspruch 1 bis 17 enthaltend einen Boratpuffer mit einem pH-Wert im Bereich von 6 bis 9 und gegebenenfalls einen Komplexbildner.

20. Zubereitungsform nach Anspruch 1 bis 17 enthaltend einen Trispuffer mit einem pH-Wert im Bereich von 6 bis 9 und gegebenenfalls einen Komplexbildner.

21. Verwendung einer Zubereitungsform nach einem der Ansprüche 1 bis 20 zur Herstellung eines Arzneimittels.

22. Verwendung einer Zubereitungsform nach einem der Ansprüche 1 bis 20 zur Herstellung eines Arzneimittels zur Behandlung von durch F XIII-Mangel charakterisierten Krankheiten.

23. Verwendung einer Zubereitungsform nach einem der Ansprüche 1 bis 20 zur Herstellung eines Arzneimittels zur Behandlung von Erkrankungen, die durch topische oder parenterale Gabe dieser Transglutaminase positiv zu beeinflussen sind.

## Claims

1. A lyophilized preparation form of a transglutaminase without any addition of foreign proteins, wherein
(i) after 6 months of storage at 4°C, the activity of the transglutaminase is still more than 80% of the starting activity prior to the lyophilization,
(ii) after the storage the lyophilizate can be dissolved readily and without any turbidity or with minimal turbidity,
(iii) as additive
(a) sugars or sugar alcohols, or mixtures thereof,
(b) histidine on its own or admixed with cysteine or isoleucine, or
(c) alanine, glutamic acid, methionine, cysteine or histidine on its own or admixed with cysteine or isoleucine admixed with sugar or sugar alcohols or mixtures thereof.

2. The preparation form as claimed in claim 1 which additionally comprises surface-active agents and/or reducing agents.

3. The preparation form as claimed in claim 1 or 2, wherein the transglutaminase is factor XIII (F XIII), or biologically active fragments, derivatives or muteins thereof.

4. The preparation form as claimed in claim 3, wherein the F XIII is F XIII from plasma, placenta, thrombocytes or macrophages/monocytes or is rF XIII, or is biologically active fragments, derivatives or muteins thereof.

5. The preparation form as claimed in one of claims 1 to 4, wherein the sugar or sugar alcohol is sucrose, maltose, trehalose, lactose, sorbitol or mannitol, or their derivatives, homologs or mixtures.

6. The preparation form as claimed in claim 1, wherein the sugars or sugar alcohols are present in combination with the amino acid His, Glu, Ile and/or Ala.

7. The preparation form as claimed in claim 2, wherein the surface-active agent is Tween 80, Tween 20, PEG, cetyl alcohol, PVP, PVA, lanolin alcohol or sorbitan monooleate.

8. The preparation form as claimed in claim 2, wherein the reducing agent is Cys, N-acetyl-Cys, thioglycerol, sodium sulfide or glutathione or mixtures thereof, where appropriate in the presence of a complexing agent.

9. The preparation form as claimed in claim 2, which comprises, as additives in addition to (an) amino acid(s), a sugar or sugar alcohol and a surface-active substance.

10. The preparation form as claimed in claim 9, which comprises, as additives, His/Tween 80/sucrose, His/Tween 20/sucrose, His/PEG/sucrose or His/Ile/PEG/sucrose.

11. The preparation form as claimed in claim 2, which comprises, as additives, (an) amino acid(s) and/or a sugar or sugar alcohol, a surface-active substance and a reducing agent.

12. The preparation form as claimed in claim 11, which comprises, as additives, (an) amino acid(s)/Cys/PEG/sucrose, (an) amino acid(s)/N-acetyl-Cys/PEG/sucrose, (an) amino acid(s)/thioglycerol/PEG/sucrose or sugar/reducing agent/PEG, where appropriate in the presence of a complexing agent.

13. The preparation form as claimed in one of claims 1 to 12, wherein the concentration of the transglutaminase is in the range from 0.003 to 50 mg/ml.

14. The preparation form as claimed in one of claims 1 to 13, wherein the concentration of the amino acids, their salts, derivatives or homologs is in the range from 0.01 to 10% (wt/vol), preferably from 0.1 to 3% (wt/vol).

15. The preparation form as claimed in one of claims 1 to 14, wherein the concentration of the sugar or sugar alcohol is between 0.1 and 20% (wt/ vol), preferably between 0.2 and 10% (wt/vol).

16. The preparation form as claimed in one of claims 2 to 15, wherein the concentration of the surface-active agent is between 0.00001 and 5% (wt/ vol), preferably between 0.0002 and 0.1% (wt/vol).

17. The preparation form as claimed in one of claims 2 to 16, wherein the concentration of the reducing agent is between 0.001% and 2% (wt/vol), preferably between 0.005 and 0.5% (wt/vol).

18. The preparation form as claimed in one of claims 1 to 17, wherein the pH is in a range from 6 to 9, preferably between 7 and 8.

19. The preparation form as claimed in claims 1 to 17, which comprises a borate buffer having a pH in the range from 6 to 9 and, where appropriate, a complexing agent.

20. The preparation form as claimed in claims 1 to 17 which comprises a Tris buffer having a pH in the range from 6 to 9 and, where appropriate, a complexing agent.

21. The use of a preparation form as claimed in one of claims 1 to 20 for preparing a pharmaceutical.

22. The use of a preparation form as claimed in one of claims 1 to 20 for preparing a pharmaceutical for treating diseases which are **characterized by** F XIII deficiency.

23. The use of a preparation form as claimed in one of claims 1 to 20 for preparing a pharmaceutical for treating diseases which can be positively influenced by the topical or parenteral administration of this transglutaminase.

## Revendications

1. Formulation lyophilisée d'une transglutaminase sans addition de protéines étrangères, dans laquelle :
i) au bout de 6 mois de stockage à 4°C, l'activité de la transglutaminase est encore de plus de 80% de l'activité initiale avant lyophilisation,
ii) le lyophilisat après le stockage est bien soluble et sans trouble ou avec un trouble minimal,
iii) la préparation contient à titre d'additifs :
a) des sucres ou des alcools de sucres, ou des mélanges de tels composés ;
b) de l'histidine, seule ou en mélange avec de la cystéine ou de l'isoleucine ; ou
c) de l'alanine, de l'acide glutamique, de la méthionine, de la cystéine ou de l'histidine, seuls ou en mélange avec de la cystéine ou de l'isoleucine en mélange avec des sucres ou des alcools de sucres ou des mélanges de ces composés.

2. Formulation selon la revendication 1, qui contient en outre des agents tensioactifs et/ou des agents réducteurs.

3. Formulation selon la revendication 1 ou 2, dans laquelle la transglutaminase est le facteur XIII (F XIII) un de ses fragments biologiquement actifs, ou bien un de ses dérivés ou mutéines.

4. Formulation selon la revendication 3, dans laquelle la transglutaminase du F XIII de plasma, de placenta, de thrombocytes, de macrophages/monocytes ou du rF XIII ou de leurs fragments biologiquement actifs, dérivés ou mutéines.

5. Formulation selon l'une quelconque des revendications 1 à 4, dans laquelle le sucre ou l'alcool de sucre est le saccharose, le maltose, le tréhalose, le lactose, le sorbitol ou le mannitol, ou bien d'un de leurs dérivés, homologues ou mélanges.

6. Formulation selon la revendication 1, dans laquelle les sucres ou les alcools de sucres sont présents en combinaison avec les acides aminés His, Glu, Ile et/ou Ala.

7. Formulation selon la revendication 2, dans laquelle l'agent tensioactif est le Tween 80, le Tween 20, le PEG, l'alcool cétylique, le PVP, le PVA, l'alcool de lanoline ou le monooléate de sorbitol.

8. Formulation selon la revendication 2, dans laquelle l'agent réducteur est le Cys, le N-acétyl-Cys, la thioglycérine, le sulfure de sodium, le glutathion ou leurs mélanges, éventuellement en présence d'un agent complexant.

9. Formulation selon la revendication 2, qui contient comme additifs, en dehors d'un ou plusieurs acides aminés, un sucre ou un alcool de sucre et une substance tensioactive.

10. Formulation selon la revendication 9, qui contient comme additifs un système His/Tween 80/saccharose, His/Tween 20/-saccharose, His/PEG/saccharose ou His/Ile/PEG/-saccharose.

11. Formulation selon la revendication 2, qui contient comme additifs un ou des acides aminés et/ou un sucre ou un alcool de sucre, une substance tensioactive et un agent réducteur.

12. Formulation selon la revendication 11, qui contient comme additifs un système acide(s) aminé(s)/Cys/PEG/saccharose, acide(s) aminé(s)/N-acétyl-Cys/PEG/saccharose, acide(s) aminé(s) /thioglycérine/PEG/saccharose ou sucre/agent réducteur /PEG, éventuellement en présence d'un agent complexant.

13. Formulation selon l'une quelconque des revendications 1 à 12, dans laquelle la concentration de la transglutaminase se situe dans la plage de 0,003 à 50 mg/ml.

14. Formulation selon l'une quelconque des revendications 1 à 13, dans laquelle la concentration des acides aminés, de leurs sels, dérivés ou homologues se situe dans la plage de 0,01% à 10% (en poids/volume), de préférence de 0,1% à 3% (en poids/volume).

15. Formulation selon l'une quelconque des revendications 1 à 14, dans laquelle la concentration du sucre ou de l'alcool de sucre se situe entre 0,1% et 20% (en poids/volume), de préférence entre 0,2% et 10% (en poids/volume).

16. Formulation selon l'une quelconque des revendications 2 à 15, dans laquelle la concentration de l'agent tensioactif se situe entre 0,00001% et 5% (en poids/volume), de préférence entre 0,0002% et 0,1% (en poids/volume).

17. Formulation selon l'une quelconque des revendications 2 à 16, dans laquelle la concentration de l'agent réducteur se situe entre 0,001% et 2% (en poids/volume), de préférence entre 0,005% et 0,5% (en poids/volume).

18. Formulation selon l'une quelconque des revendications 1 à 17, dans laquelle la valeur du pH se situe dans une plage de 6 à 9, de préférence entre 7 et 8.

19. Formulation selon les revendications 1 à 17, contenant un tampon de borate avec une valeur du pH dans la plage de 6 à 9 et éventuellement un agent complexant.

20. Formulation selon les revendications 1 à 17, contenant un tampon de Tris avec une valeur du pH dans la plage de 6 à 9 et éventuellement un agent complexant.

21. Utilisation d'une forme de préparation selon l'une quelconque des revendications 1 à 20 pour la fabrication d'un médicament.

22. Utilisation d'une formulation selon l'une quelconque des revendications 1 à 20 pour la fabrication d'un médicament pour le traitement de maladies **caractérisées par** une carence en F XIII.

23. Utilisation d'une forme de préparation selon l'une quelconque des revendications 1 à 20 pour la fabrication d'un médicament pour le traitement d'affections qui peuvent être influencées positivement par administration topique ou parentérale de cette transglutaminase.
